# EUROPEAN PATENT SPECIFICATION

(11) **EP 3 685 700 B1**
(45) Date of publication and mention of the grant of the patent: **17.01.2024**
(21) Application number: 18859887.4
(22) Date of filing: 30.08.2018
(51) Int. Cl.: A45D 44/22, A61K 9/70, A45D 44/00, A61K 8/02, D04H 13/00

(54) **COSMETIC SHEET AND COSMETIC ARTICLE**
KOSMETIKFOLIE UND KOSMETIKARTIKEL
FEUILLE COSMÉTIQUE ET ARTICLE COSMÉTIQUE

(30) Priority: 19.09.2017 JP 2017178544
(43) Date of publication of application: 29.07.2020
(73) Proprietor: Taiki Corp., Ltd., Osaka-shi, Osaka 534-0014 (JP)
(72) Inventor: HARA, Yuka, Osaka-shi, Osaka 534-0014 (JP); DOI, Yukiko, Osaka-shi, Osaka 534-0014 (JP); NAKAMURA, Koji, Osaka-shi, Osaka 534-0014 (JP)
(74) Representative: Schön, Christoph
(86) International application number: PCT/JP2018/032068
(87) International publication number: WO 2019/058915

(56) References cited:
- WO-A1-2015/198933
- WO-A1-2016/103449
- JP-A- H11 137 338
- JP-A- S60 111 603
- JP-A- 2003 135 146
- JP-A- 2013 512 154
- JP-A- 2014 024 764
- JP-A- 2015 223 506
- JP-A- 2017 105 837
- JP-B2- 3 866 555
- JP-U- 3 036 480
- JP-U- 3 080 940
- US-A1- 2016 107 004

## Description

### TECHNICAL FIELD

The present invention relates to a cosmetic sheet and a cosmetic article. More specifically, the present invention relates to, for example, a cosmetic sheet such as a face mask, a foot care sheet or a moisturizing sheet, and a cosmetic article in which the cosmetic sheet is accommodated.

### BACKGROUND ART

In recent years, as a face mask including a liquid-impregnated substrate, in which the liquid-impregnated substrate containing a cosmetic liquid can be easily spread out, separation and removal of the cosmetic liquid from the liquid-impregnated substrate are suppressed, and evaporation of an active ingredient included in the liquid-impregnated substrate is also suppressed, a face mask including a liquid-blocking film and a liquid-impregnated substrate containing a cosmetic liquid, in which the liquid-impregnated substrate is superposed on the liquid-blocking film, and bonded to the liquid-blocking film in the form of spots has been proposed (for example, see paragraphs [0008] and [0009] of Patent Literature 1). As a substrate used in the liquid-impregnated substrate of the above-mentioned face mask, a nonwoven fabric made of rayon fibers, cotton fibers, polyester fibers and the like have been used. The above-mentioned nonwoven fabric has been fused into one body with the liquid-blocking film by including a thermally fusible fiber in the nonwoven fabric, or applying a hot-melt binder to the nonwoven fabric (for example, see paragraph [0014] of Patent Literature 1). As a face mask for preventing evaporation of moisture and imparting moisture feeling, a face mask including a nonwoven fabric made of cotton fibers and a resin film made of polyethylene, in which the nonwoven fabric is united into one body with the resin film has been proposed (for example, see claim 1 of Patent Literature 2).

However, any of the above-mentioned face masks are small in stretching property, and the film is possibly removed from the nonwoven fabric when the face mask is extended to 50% from its original length, because the nonwoven fabric does not follow the extended film.

As a cosmetic sheet, which is excellent in stretching property and followability of a nonwoven fabric to a film, which is excellent in lift-up property, that is, excellent in property for lifting up a cheek and a face line of a human face when the face mask is applied to the face, and which can be suitably used in, for example, a face mask and the like, a cosmetic sheet including a nonwoven fabric and a film laminated on the nonwoven fabric, in which a nonwoven fabric containing a thermally fusible fiber is used as the above-mentioned nonwoven fabric, a film containing a thermoplastic elastomer is used as the above-mentioned film, and the thermally fusible fiber contained in the nonwoven fabric is fused into one body with the film has been proposed (for example, see Patent Literature 3).

The above-mentioned cosmetic sheet is excellent in stretching property and followability of the nonwoven fabric to the film, and also excellent in lift-up property. Therefore, the cosmetic sheet can be suitably used in, for example, a face mask, a foot care sheet, a moisturizing sheet and the like.

In the above-mentioned cosmetic sheet, the film containing a thermoplastic elastomer is used on its surface, and the film has adhesiveness. Accordingly, the cosmetic sheet is poor in processability when the cosmetic sheet is cut to have a predetermined shape with a cutting blade, because the cosmetic sheet sometimes adheres to the cutting blade due to its adhesiveness. In addition, when the above-mentioned cosmetic sheet is accommodated in a storage container for accommodating a cosmetic sheet, the above-mentioned film adheres to an inner surface of the storage container. Accordingly, a user sometimes cannot easily take out the cosmetic sheet from the storage container.

### PRIOR ART LITERATURES

### PATENT LITERATURES

Patent Literature 1: Japanese patent No. 3866555
Patent Literature 2: Japanese utility model registration No. 3080940
Patent Literature 3: International publication No. WO 2015/198933 A1

### SUMMARY OF THE INVENTION

### PROBLEMS TO BE SOLVED BY THE INVENTION

The present invention has been made in view of the above-mentioned prior art. An object of the present invention is to provide a cosmetic sheet which hardly adheres to a member such as a cutting blade or a storage container when the cosmetic sheet is contacted with the member, and a cosmetic article having the cosmetic sheet.

### MEANS FOR SOLVING THE PROBLEMS

The present invention relates to:
(1) a cosmetic sheet including a nonwoven fabric having stretching property, an elastomer film and a release layer, wherein
   the elastomer film is laminated on the nonwoven fabric, and fibers included in the above-mentioned nonwoven fabric are bonded to the elastomer film, and the above-mentioned release layer is formed by applying and drying a mold releasing agent on the surface of the above-mentioned elastomer film;
      and
   the release layer is formed from at least one mold releasing agent selected from the group consisting of a silicone resin-based mold releasing agent, a urethane resin-based mold releasing agent, a wax-based mold releasing agent and a fluorocarbon resin-based mold releasing agent;
(2) the cosmetic sheet according to the above-mentioned item (1), wherein the above-mentioned nonwoven fabric is a nonwoven fabric containing thermally fusible fibers; the above-mentioned elastomer film is an elastomer film containing a thermoplastic elastomer; and the above-mentioned thermally fusible fibers are fused into one body with the above-mentioned elastomer film;
(3) a cosmetic article including the above-mentioned cosmetic sheet according to any one of the above-mentioned items (1) to (2), and a storage container for accommodating the cosmetic sheet, wherein the above-mentioned cosmetic sheet is accommodated in the storage container;
(4) the cosmetic article according to the above-mentioned item (3), wherein the above-mentioned cosmetic sheet is folded so that the release layer of the cosmetic sheet is positioned at an inner surface of the folded cosmetic sheet; and
(5) the cosmetic article according to the above-mentioned item (3) or (4), wherein the cosmetic sheet is folded so that the release layer of the above-mentioned cosmetic sheet is contacted with an inner surface of the storage container.

### EFFECTS OF THE INVENTION

According to the present invention, there are provided a cosmetic sheet which hardly adheres to a member such as a cutting blade or a storage container when the cosmetic sheet is contacted with the member, and a cosmetic article having the cosmetic sheet.

### BRIEF DESCRIPTION OF THE DRAWINGS

Fig. 1 is a schematic sectional view illustrating one embodiment of a cosmetic sheet of the present invention.
Fig. 2 is a schematic explanation view showing one embodiment of orders for folding the cosmetic sheet of the present invention and one embodiment of orders for accommodating the cosmetic sheet in a storage container.
Fig. 3 is a schematic explanation view showing another embodiment of orders for folding the cosmetic sheet of the present invention, and another embodiment of orders for accommodating the cosmetic sheet in the storage container.

### MODE FOR CARRYING OUT THE INVENTION

As mentioned above, the cosmetic sheet of the present invention includes an elastic nonwoven fabric, an elastomer film and a release layer, a fiber contained in the above-mentioned nonwoven fabric is bonded to the above-mentioned elastomer film, and the above-mentioned release layer is formed on the surface of the above-mentioned elastomer film as it is defined in the appended claims.

According to the cosmetic sheet of the present invention, since the surface of the adhesive elastomer film is covered with the release layer, the cosmetic sheet exhibits excellent properties such that the cosmetic sheet is hard to adhere to a member such as a cutting blade or a storage container when the cosmetic sheet is contacted with the member. Accordingly, the cosmetic sheet of the present invention is excellent in processability, for example, when the cosmetic sheet is cut to have a predetermined shape. In addition, the cosmetic sheet of the present invention can be easily taken out from the storage container when the cosmetic film is accommodated in the storage container for the cosmetic sheet, since the cosmetic sheet is prevented from adhesion to the inner surface of the storage container.

The nonwoven fabric used in the present invention has stretching property. The nonwoven fabric having stretching property is a sheet-like fabric in which fibers or filaments are irregularly arranged, and is one other than a woven fabric and a knitted fabric. The nonwoven fabric mainly has a role for holding a chemical solution, a cosmetic and the like.

The fiber which constitutes the nonwoven fabric include a thermally fusible fiber and a thermally infusible fiber. The thermally fusible fiber and the thermally infusible fiber can be used alone respectively or in combination.

The thermally fusible fiber includes, for example, polyolefin fibers such as a polyethylene fiber and a polypropylene fiber; polyester fibers such as a polyethylene terephthalate fiber and a polybutylene terephthalate fiber; acrylic fibers such as a polymethyl methacrylate fiber; a polyurethane fiber; a polyvinyl alcohol fiber; a polystyrene fiber; a polyamide fiber represented by various nylon; a polycarbonate fiber; an ethylene-vinyl acetate fiber; a vinyl acetate-vinyl chloride fiber, and the like, and also includes a polyester-polyester composite fiber, a polyester-polyolefin composite fiber, a polyester-polyamide composite fiber, a polyamide-polyamide composite fiber, and the like. The present invention is not limited only to those exemplified ones. These thermally fusible fibers can be used alone respectively or in combination of two or more kinds thereof. The composite fiber includes, for example, a sheath-core type conjugate fiber, a side by side type conjugate fiber and the like, and the present invention is not limited only to those exemplified ones.

The thermally infusible fiber includes, for example, a rayon fiber, a cotton fiber, a pulp fiber, a linen fiber, an aromatic polyamide fiber, a phenol resin fiber, a carbon fiber, a ceramic fiber, a metal fiber and the like, and the present invention is not limited only to those exemplified ones. These thermally infusible fibers can be used alone respectively or in combination of two or more kinds thereof. Among these thermally infusible fibers, the rayon fiber, the cotton fiber and the pulp fiber are preferred from the viewpoint of imparting of a proper strength and proper stretching property to the cosmetic sheet of the present invention.

The fiber length and fineness of the fiber which constitutes the nonwoven fabric are not particularly limited. The fiber length of the fiber which constitutes the nonwoven fabric is preferably 20 to 100 mm or so, and more preferably 40 to 80 mm or so. The fineness of the fiber which constitutes the nonwoven fabric is preferably 1 to 30 deniers or so, and more preferably 3 to 10 deniers or so. The fiber which constitutes the nonwoven fabric may include, for example, a short fiber having a fiber length of preferably 0.5 to 20 mm or so, and more preferably 1 to 15 mm or so within a scope which would not hider an object of the present invention.

When the fiber included in the nonwoven fabric is bonded to the above-mentioned elastomer film with the aid of a binder (adhesive agent), as the fiber included in the nonwoven fabric, the thermally fusible fiber can be used alone, the thermally infusible fiber can be used alone, or the thermally fusible fiber and the thermally infusible fiber can be used together in an arbitrary ratio.

When the fiber included in the nonwoven fabric and the above-mentioned elastomer film are bonded into one body by thermal fusion, it is preferred to use the thermally fusible fiber alone or a combination of the thermally fusible fiber and the thermally infusible fiber as the fiber included in the nonwoven fabric. In this case, the content of the thermally fusible fiber in the nonwoven fabric is preferably 10% by mass or more, more preferably 15% by mass or more, and furthermore preferably 20% by mass or more, from the viewpoint of firm fusional bonding of the nonwoven fabric and the elastomer film, and its upper limit is 100% by mass. In addition, the content of the thermally infusible fiber in the nonwoven fabric is preferably 90% by mass or less, more preferably 85% by mass or less, furthermore preferably 80% by mass or less, from the viewpoint of firm fusional bonding of the nonwoven fabric and the elastomer film, and its lower limit is 0% by mass.

The nonwoven fabric can be produced, for example, by entangling fibers included in the nonwoven fabric in a manner such as a mechanical method such as a needle punching method, an air-entangling method, a water-jet entangling method, a wet papermaking method or a high-pressure vapor entangling method, or a thermal fusion method such as a spun-bonding method or a melt-blowing method, or by bonding the fibers included in the nonwoven fabric in a manner such as a method for bonding using a binder, and the like.

The area weight of the nonwoven fabric cannot be absolutely determined because the area weight varies depending on uses of the cosmetic sheet of the present invention. The area weight is preferably 5 to 200 g/m², more preferably 10 to 180 g/m², furthermore preferably 20 to 150 g/m², and even more preferably 30 to 100 g/m², from the viewpoint of imparting of proper strength and proper stretching property to the cosmetic sheet of the present invention.

It is preferred that the nonwoven fabric is stretchable in at least one direction from the viewpoint of pulling up a cheek and a face lines of a human face with a proper force, and maintaining of its lifted state when the cosmetic sheet of the present invention is used in, for example, a face mask and the like.

The direction of elongation of the nonwoven fabric generally includes a longitudinal direction and a lateral direction. The longitudinal direction of elongation of the nonwoven fabric usually means a flow direction of the nonwoven fabric when the nonwoven fabric is produced. The lateral direction of elongation of the nonwoven fabric usually means a direction perpendicular to the flow direction of the nonwoven fabric. When the above-mentioned nonwoven fabric is used in, for example, a face mask and the like, it is preferred that a nonwoven fabric which is easily stretched and contracted in at least one direction is used, and that the lateral direction of the nonwoven fabric matches with the lateral direction of a face of a person from the viewpoint that a cheek and a face line of a person are lifted up by an adequate force, and that its lifted state is maintained.

Each of tensile elongations in the longitudinal direction and lateral direction of the nonwoven fabric is preferably 40 to 110% (longitudinal direction) and 120 to 220% (lateral direction) respectively, and more preferably 45 to 105% (longitudinal direction) and 125 to 215% (lateral direction) respectively, from the viewpoint that a cheek and a face line of a person are lifted up by an adequate force, and that its lifted state is maintained when the cosmetic sheet of the present invention is used in, for example, a face mask and the like.

Incidentally, the tensile elongation of the nonwoven fabric is a value as determined in accordance with the item "6.3.1" of JIS L1913 (2010). More specifically, the tensile elongation in each of the longitudinal direction and lateral direction of the nonwoven fabric is a value as determined by cutting out three rectangular test pieces having a size of 50 mm × 150 mm from the nonwoven fabric, setting each of the test pieces so that an initial length (length of the test piece) is 100 mm in a tensile tester, stretching the test piece at a tensile speed 300 mm/min, determining an elongation when the test piece is broken, calculating a tensile elongation (ratio of a length after elongation to an original length), and calculating an average of the tensile elongations of the three test pieces in each of the longitudinal direction and the transverse direction of the nonwoven fabric.

In addition, a ratio of the tensile elongation in the lateral direction of the nonwoven fabric to the tensile elongation in the vertical direction of the nonwoven fabric (tensile elongation in the lateral direction / the value of the tensile elongation in the longitudinal direction) is preferably from 1 to 5, more preferably from 1.5 to 3.5, and furthermore preferably from 2 to 3, from the viewpoint that a cheek and a face line of a person are lifted up by an adequate force, and that its lifted state is maintained when the cosmetic sheet of the present invention is used in, for example, a face mask and the like.

The non-woven fabric which is easily stretched and contracted in at least one direction includes, for example, a product commercially available from Daiwabo Polytec Co., Ltd. under the product number of DFS (SH) R5-50 (tensile elongation in longitudinal direction: 70 ± 25%, tensile elongation in lateral direction: 170 ± 45%), a product commercially available from Daiwabo Polytec Co., Ltd. under the product number of DFS (SH) R5-70 (tensile elongation in longitudinal direction: 80 ± 25%, tensile elongation in the lateral direction: 170 ± 45%) and the like, and the present invention is not limited only to those exemplified ones.

The elastomer film is laminated on the nonwoven fabric, and the fibers included in the nonwoven fabric are bonded to the elastomer film. The elastomeric film mainly makes a role for preventing a chemical liquid, a cosmetic ingredient and the like included in the cosmetic sheet of the present invention from evaporating.

The elastomer used as a raw material of the above-mentioned elastomer film includes a thermoplastic elastomer and a thermosetting elastomer. The thermoplastic elastomer and the thermosetting elastomer can be used alone respectively or in combination. As the above-mentioned elastomer, it is preferred that the thermoplastic elastomer is used solely or in combination with the thermosetting elastomer, and it is more preferred that the thermoplastic elastomer is used solely, from the viewpoint of production of the cosmetic sheet which is excellent in stretching property and followability of the nonwoven fabric to the elastomer film.

The above-mentioned thermoplastic elastomer includes, for example, polyolefin-based elastomers such as polyethylene elastomer, polypropylene elastomer, ethylene-propylene elastomer and ethylene-octene elastomer, polyurethane elastomer, polystyrene elastomer, polyester elastomer, polyamide elastomer, vinyl chloride resin elastomer and the like, and the present invention is not limited only to those exemplified ones. These elastomers can be used alone respectively, or at least two kinds thereof can be used in combination. Among the elastomers, the polyolefin-based elastomer is preferable, and polyethylene elastomer, ethylene-propylene elastomer and ethylene-octene elastomer are more preferable, and polyethylene elastomer and ethylene-octene elastomer are furthermore preferable, from the viewpoint of production of the cosmetic sheet which is excellent in stretching property, followability of the nonwoven fabric to the elastomer film and lift-up property. These polyolefin-based elastomers can be used alone respectively or in combination.

The above-mentioned thermoplastic elastomer can be used in combination with a thermoplastic resin as occasion demands. The thermoplastic resin includes, for example, polyolefin-based resins such as polyethylene such as low density polyethylene, medium density polyethylene and high density polyethylene, polypropylene, ethylene-propylene copolymer, ethylene-butene copolymer, ethylene-pentene copolymer, ethylene-hexene copolymer, propylene-butene copolymer, propylene-pentene copolymer, propylene-hexene copolymer, ethylene-vinyl acetate copolymer and ethylene-(meth)acrylic acid copolymer; styrene-based resins such as polystyrene and acrylonitrile-butadiene-styrene copolymer (ABS resin); polyamide-based resins such as 6-nylon, 66-nylon and 12-nylon; polyamide-imide; polyurethanes; polyimides; polyetherimides; acrylic resins; vinyl chloride resins; polyester-based resins such as polyethylene terephthalate and polybutylene terephthalate; polycarbonates such as bisphenol A-based polycarbonates; and the like. The present invention is not limited only to those exemplified ones. These thermoplastic resins can be used alone respectively, or at least two kinds thereof can be used in combination. Among the above-mentioned thermoplastic resins, the polyolefin-based resins such as polyethylene and polypropylene are preferable, and low-density polyethylene is more preferable, from the viewpoint of imparting of appropriate strength and appropriate stretching property to the cosmetic sheet of the present invention.

When the above-mentioned thermoplastic elastomer and the above-mentioned thermoplastic resin are used in combination, the content of the thermoplastic elastomer in the elastomer film is preferably 10% by mass or more, more preferably 20% by mass or more, furthermore preferably 30% by mass or more, still more preferably 40% by mass or more, from the viewpoint of production of the cosmetic sheet which is excellent in stretching property and followability of the nonwoven fabric to the elastomer film, and which is also excellent in lift-up property. The content of the thermoplastic resin in the above-mentioned elastomer film is preferably 90% by mass or less, more preferably 80% by mass or less, furthermore preferably 70% by mass or less, still more preferably 60% by mass or less, from the viewpoint of production of the cosmetic sheet which is excellent in stretching property and followability of the nonwoven fabric to the elastomer film, and which is also excellent in lift-up property. In addition, the content of the thermoplastic elastomer in the above-mentioned elastomer film is preferably 100% by mass or less, more preferably 95% by mass or less, and furthermore preferably 90% by mass or less, from the viewpoint of production of the cosmetic sheet which is excellent in stretching property and followability of the nonwoven fabric to the elastomer film, and which is also excellent in lift-up property. The content of the thermoplastic elastomer in the above-mentioned elastomer film is preferably 0% by mass or more, more preferably 5% by mass or more, and furthermore preferably 10% by mass or more, from the viewpoint of production of the cosmetic sheet which is excellent in stretching property and followability of the nonwoven fabric to the elastomer film, and which is also excellent in lift-up property.

The above-mentioned thermosetting elastomer includes, for example, diene-based synthetic rubbers such as polyisoprene rubber, polybutadiene rubber, styrene-butadiene rubber, polychloroprene rubber, nitrile rubber and ethylene-propylene rubber; non-diene-based synthetic rubbers such as ethylene-propylene rubber, butyl rubber, acrylic rubber, polyurethane rubber, fluorocarbon rubber, silicone rubber and epichlorohydrin rubber; and the like. The present invention is not limited only to those exemplified ones. These thermosetting elastomers can be used alone respectively, or at least two kinds thereof can be used in combination. Among the above-mentioned thermosetting elastomers, polyurethane rubber and silicone rubber are preferable from the viewpoint of production of the cosmetic sheet which is excellent in stretching property, followability of the nonwoven fabric to the elastomer film and lift-up property.

The elastomer film can be produced, for example, by a method which includes dissolving a raw material containing the elastomer in an organic solvent, casting the resulting solution to form a coating film, removing the organic solvent from the formed coating film; a method which includes heating the raw material containing the thermoplastic elastomer to melt, and extruding the resulting melted raw material through a T-die or the like; and the like.

The thickness of the elastomer film cannot be absolutely determined because the thickness of the elastomer film is different depending on uses of the cosmetic sheet of the present invention. The thickness of the elastomer film is preferably 1 to 50 pm, more preferably 3 to 30 pm, and furthermore preferably 5 to 15 pm, from the viewpoint of imparting of appropriate strength, appropriate flexibility and appropriate stretching property to the cosmetic sheet of the present invention.

The fibers included in the above-mentioned nonwoven fabric can be united into one body with the above-mentioned elastomer film, for example, by a method which includes using a nonwoven fabric containing a thermally fusible fiber and an elastomer film containing a thermoplastic elastomer, and fusing the thermally fusible fiber with the elastomer film to be united into one body; a method which includes bonding the fibers included in the above-mentioned nonwoven fabric to the above-mentioned elastomer film with the aid of a binder (adhesive agent) to be united into one body; and the like.

The method which includes using the above-mentioned nonwoven fabric containing a thermally fusible fiber and the elastomer film containing a thermoplastic elastomer, and fusing the thermally fusible fiber with the elastomer film to be united into one body includes, for example, a method which includes uniting an elastomer film in a thermally melted state just after the formation of the melted elastomer film by extruding from an extruder or the like, with the nonwoven fabric into one body (extrusion laminating method); a method which includes producing the nonwoven fabric and the elastomer film separately, laminating the nonwoven fabric in which the thermally fusible fibers included in the nonwoven fabric are heated to melt with the elastomer film in a melted state or an unmelted state, preferably in a melted state, and performing press bonding of both to be united into one body; and the like. The present invention is not limited only to those exemplified ones.

In the present invention, it is preferred that the fibers which constitute the nonwoven fabric are softened or melted by the heat of the elastomer film under the condition of a melted state, and the fibers which constitute the nonwoven fabric are bonded to the elastomer film. When the fibers which constitute the nonwoven fabric are bonded to the elastomer film as mentioned above, a cosmetic sheet, in which the nonwoven fabric follows the elastomer film, and separation between the nonwoven fabric and the elastomer film is hardly generated even though the cosmetic sheet is stretched, can be obtained.

When the nonwoven fabric is united into one body with the elastomer film including the thermoplastic elastomer by laminating the nonwoven fabric with the elastomer film having a thermally melted state just after extrusion molding with an extruder or the like, the cosmetic sheet of the present invention has some advantages such that the cosmetic sheet is excellent in stretching property and lift-up property.

Thus, a laminate in which the fibers included in above-mentioned nonwoven fabric are bonded to the above-mentioned elastomer film can be obtained.

Next, the release layer is formed on the surface of the elastomer film of the laminate obtained in the above. The release layer mainly makes a role for suppressing adhesion due to the elastomer film.

The release layer is formed from at least one mold releasing agent selected from the group consisting of a silicone resin-based mold releasing agent, a urethane resin-based mold releasing agent, a wax-based mold releasing agent, a fluorocarbon resin-based mold releasing agent. These mold releasing agents can be used alone respectively, or at least two kinds thereof can be used in combination. Among these mold releasing agents, the silicone resin-based mold releasing agent, the urethane resin-based mold releasing agent and the fluorocarbon resin-based mold releasing agent are preferable, and the silicone resin-based mold releasing agent and the urethane resin-based mold releasing agent are furthermore preferable, since these mold releasing agents are excellent in followability of the nonwoven fabric to the elastomer film, stretching property, affinity of the elastomer film with the release layer, and release property of the release layers with each other.

The silicone resin which is used in the silicone-based mold releasing agent includes, for example, a methyl silicone resin such as polydimethylsiloxane, a methylphenyl silicone resin, a phenyl silicone resin, an alkyd-modified silicone resin, a polyester-modified silicone resin, a urethane-modified silicone resin, an epoxy-modified silicone resin, an acryl-modified silicone resin and the like, and the present invention is not limited only to those exemplified ones. These silicone resins can be used alone respectively, or at least two kinds thereof can be used in combination.

The urethane resin which is used in the urethane resin-based mold releasing agent includes, for example, a urethane resin obtained by reacting polyvinyl alcohol with an aliphatic isocyanate, a urethane resin obtained by reacting an ethylene-vinyl alcohol copolymer with an aliphatic isocyanate, and the like. The present invention is not limited only to those exemplified ones. These urethane resins can be used alone respectively, or at least two kinds thereof can be used in combination.

Representative wax used in the wax-based mold releasing agent includes, for example, polyolefin wax such as polyethylene wax, paraffin wax, palm wax, soy wax, rice wax, sunflower wax, bees wax, montan wax, and the like. The present invention is not limited only to those exemplified ones. These waxes can be used alone respectively, or at least two kinds thereof can be used in combination.

Representative fluorocarbon resin used in the fluorocarbon resin-based mold releasing agent includes polytetrafluoroethylene and the like, and the present invention is not limited only to those exemplified ones.

A method for forming the release layer on the surface of the elastomer film of the above-mentioned laminate includes a method for applying the mold releasing agent on the surface of the above-mentioned elastomer film. A method for applying the mold releasing agent on the surface of the above-mentioned elastomer film includes, for example, coating methods such as a bar coating method and a gravure roll coating method, and the present invention is not limited only to those exemplified ones.

When the mold releasing agent is applied on the surface of the above-mentioned elastomer film, a release layer is formed by drying the mold releasing agent. A method for drying the mold releasing agent cannot be absolutely determined because the method differs depending on the kind of the mold releasing agent. Accordingly, it is preferred that the method is appropriately determined in accordance with the kind of the mold releasing agent. The method for drying the mold releasing agent includes, for example, a hot-air drying method, a natural drying method, a drying method by heating and the like, and the present invention is not limited only to those exemplified ones.

The thickness of the release layer formed as mentioned above is not particularly limited. The thickness of the release layer is preferably 0.01 µm or more from the viewpoint of enhancement in release property and increase in mechanical strength of the release layer, and is preferably 1.0 µm or less from the viewpoint of enhancement in efficiency for forming the release layer and flexibility of the cosmetic sheet.

In addition, when the cosmetic sheet of the present invention is produced by fusing the nonwoven fabric and the elastomer film containing the thermoplastic elastomer to be formed into one body, the nonwoven fabric is difficult to be separated from the elastomer film in stretching the cosmetic sheet, and the elastomer film containing the thermoplastic elastomer is excellent in stretching property. Accordingly, the cosmetic sheet is excellent in stretching property, and has excellent property such that elongation after stretching is small. Particularly, when the nonwoven fabric is laminated with the elastomer film by the extrusion laminating method, since the nonwoven fabric can be strongly bonded to the elastomer film, the nonwoven fabric becomes harder to be separated from the elastomer film, and stretching property of the elastomer film is improved. Accordingly, the cosmetic sheet of the present invention shows excellent properties such that the cosmetic sheet is excellent in stretching property and that elongation after stretching becomes smaller.

Incidentally, the basis weight of the cosmetic sheet of the present invention cannot be absolutely determined, because the basis weight differs depending on its uses and the like. The basis weight of the cosmetic sheet is preferably 10 to 250 g/m² or so, more preferably 15 to 200 g/m² or so, furthermore preferably 20 to 150 g/m² or so, and even more preferably 25 to 120 g/m² or so, from the viewpoint of production of the cosmetic sheet being excellent in stretching property and followability of the nonwoven fabric to the elastomer film, and also excellent in lift-up property.

The nonwoven fabric used in the cosmetic sheet of the present invention can be impregnated with a cosmetic as occasion demands. The cosmetic liquid includes, for example, a cosmetic liquid containing a component for imparting softness to a skin, a cosmetic liquid containing a component for imparting moisture retaining property, a cosmetic liquid containing a nutrient component, a cosmetic liquid containing a medicinal ingredient, and the like. The present invention is not limited only to those exemplified ones. Embodiments of the form of the cosmetic liquid include, for example, an aqueous solution, an emulsion, an oil, a cream and the like, and the present invention is not limited only to those exemplified ones.

Uses of the cosmetic sheet of the present invention include, for example, a face mask, a sheet for supplying cosmetics to various places of a human body, a foot care sheet, a moisturizing sheet, a sheet for a poultice sheet and the like, and the present invention is not limited only to those exemplified ones.

Hereinafter, one embodiment of the cosmetic sheet of the present invention and one embodiment of the cosmetic article of the present invention are more specifically described based on drawings. However, the present invention is not limited to each embodiment.

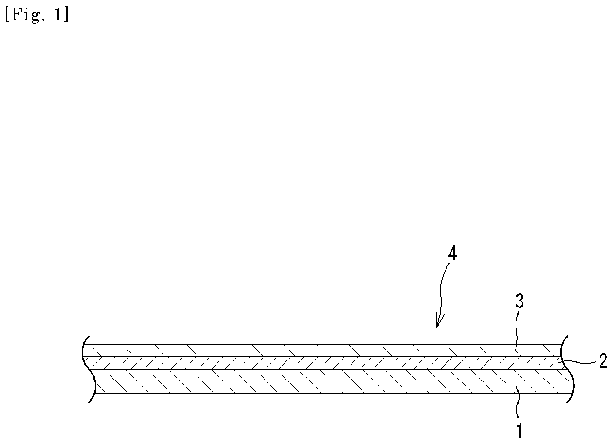

Fig. 1 is a schematic sectional view showing one embodiment of the cosmetic sheet of the present invention. The cosmetic sheet 4 shown in Fig. 1 has a stretchable nonwoven fabric 1, an elastomer film 2 and a release layer 3. The fibers (not shown in the figure) included in the nonwoven fabric 1 are bonded to the elastomer film 2, and the release layer 3 is formed on the surface of the elastomer film 2. The cosmetic sheet 4 of the present invention can have the above-mentioned basic structure, and another layer (not shown in the figure) or another film (not shown in the figure) can be intermediated between the non-woven fabric 1 and the elastomer film 2 within a scope which would not hinder an object of the present invention. Alternatively, a surface layer (not shown in the figure) such as a resin layer, a film, or a fibrous layer such as a woven fabric or a nonwoven fabric can be formed on the surface of the release layer 3.

The cosmetic article of the present invention has the cosmetic sheet and a storage container for storing the cosmetic sheet, and can be characterized in that the above-mentioned cosmetic sheet is accommodated in the storage container. Since the cosmetic article of the present invention has the above-mentioned constituents, the cosmetic article has some advantages such that the above-mentioned cosmetic sheet is hard to be adhered to a member such as an inner surface of the above-mentioned container when the above-mentioned cosmetic sheet is contacted with the member.

In addition, in the cosmetic article of the present invention, it is preferred that the above-mentioned cosmetic sheet is folded so that the release layer of the cosmetic sheet is inside.

A conventional cosmetic sheet necessitates complicated procedures which include folding the cosmetic sheet so that an elastomer film of the cosmetic sheet is inside, and putting a separator inside the folded cosmetic sheet in order to prevent the elastomer film from contacting with each other, and thereafter accommodating the cosmetic sheet in a storage container for the cosmetic sheet. Accordingly, the cosmetic sheet has a defect in that productivity of the cosmetic sheet is low. Moreover, according to the conventional cosmetic sheet, since there is a necessity to dispose of an unnecessary separator which is taken out from a cosmetic article in which the cosmetic sheet is accommodated, the cosmetic sheet is inconvenient for a user, and the dispose of the unnecessary separator is unfavorable from the viewpoint of reduction of the amount of wastes for global environment protection.

In addition, in the cosmetic article of the present invention, when the cosmetic sheet is folded so that the release layer of the above-mentioned cosmetic sheet is contacted with an inner surface of the above-mentioned storage container, the cosmetic sheet can be hard to be adhered to a member such as an inner surface of the above-mentioned storage container in a case where the cosmetic sheet is contacted with the member.

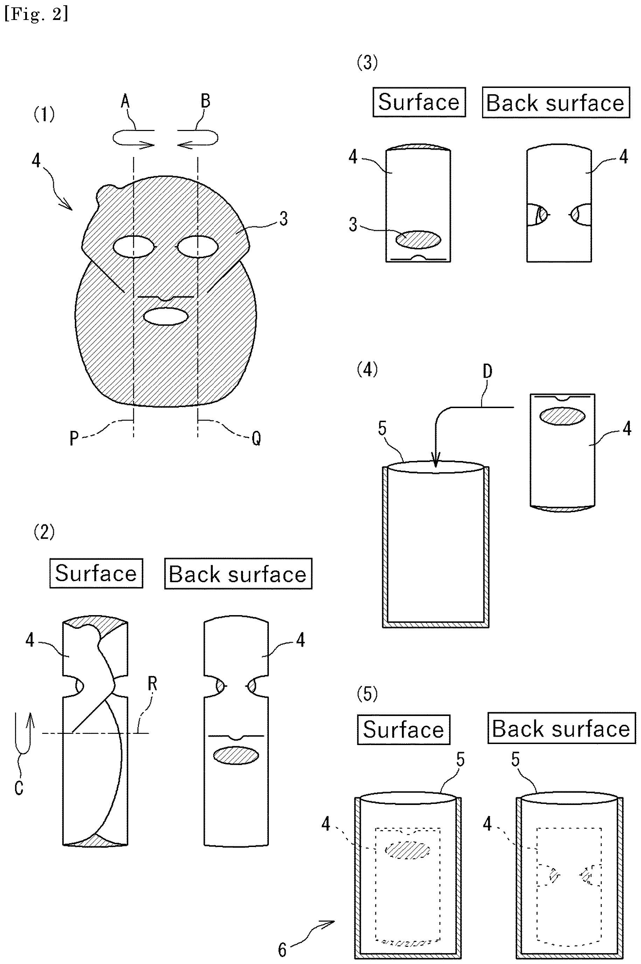

The cosmetic sheet of the present invention can be folded, for example, in accordance with orders shown in Fig.2 (1) to Fig.2 (3). According to the cosmetic article of the present invention, the cosmetic sheet can be accommodated in the storage container, for example, in accordance with orders shown in Fig.2 (4) and Fig.2 (5).

Incidentally, Fig. 2 is a schematic explanation view showing one embodiment of orders for folding the cosmetic sheet of the present invention and one embodiment of orders for accommodating the cosmetic sheet in a storage container. The cosmetic sheet 4 shown in Fig. 2 has a shape of a face mask.

In Fig. 2, the release layer 3 of the cosmetic sheet 4 of the present invention is shown by oblique lines. First, as shown in Fig. 2 (1), the cosmetic sheet 4 of the present invention is valley folded at the creases P, Q as shown by arrows A and B, respectively, to fold the cosmetic sheet 4 as shown in Fig. 2 (2). The folded cosmetic sheet 4 shown in Fig. 2 (2) is then valley folded at the crease R as shown by an arrow C, to fold the cosmetic sheet 4 as shown in Fig. 2 (3). In accordance with the above orders, the cosmetic sheet 4 of the present invention can be folded so that the release layer 3 is positioned at an inner surface of the folded cosmetic sheet 4.

The cosmetic sheet 4 of the present invention folded in the above is accommodated in the storage container 5 of the cosmetic sheet 4, for example, as shown by an arrow D of Fig. 2 (4), and thereby the cosmetic article 6 can be obtained as shown in Fig. 2 (5). Incidentally, after the cosmetic sheet 4 is accommodated in the storage container 5, an opening of the storage container 5 of the cosmetic sheet 4 can be sealed by, for example, thermal fusion or the like.

The storage container 5 of the cosmetic sheet 4 includes, for example, a bag made of a polyester film in which aluminum vapor deposition is applied to an inner surface thereof, an aluminum pouch and the like, and the present invention is not limited only to those exemplified ones. In addition, the size of the storage container 5 of the cosmetic sheet 4 is not particularly limited, and the size can be one having a size suitably for accommodating the cosmetic sheet 4 of the present invention.

According to the embodiments of the orders for folding the cosmetic sheet 4 and the embodiments of orders for accommodating the cosmetic sheet 4 in the storage container 5, which are shown in Fig. 2, the cosmetic sheet 4 is folded so that the nonwoven fabric of the cosmetic sheet 4 is contacted with an inner surface of the storage container 5. Accordingly, the cosmetic sheet 4 can be prevented from adhering to the inner surface of the storage container 5. In addition, a part of the release layer of the cosmetic sheet 4 might be exposed to the opening of the cosmetic sheet 4, to be contacted with the inner surface of the storage container 5. However, the release layer of the cosmetic sheet 4 can prevent the elastomer film of the cosmetic sheet 4 from adhering to the inner surface of the storage container 5. Accordingly, a user of the cosmetic sheet 4 can easily take out the cosmetic sheet 4 from the storage container 5.

In addition, according to the embodiments shown in Fig. 2, since the cosmetic sheet 4 is folded so that the release layer 3 of the cosmetic sheet 4 is positioned at the inner surface of the folded cosmetic sheet 4, the cosmetic sheet 4 does not necessitate a separator. Accordingly, the cosmetic sheet 4 and the cosmetic article 6 of the present invention have some advantageous merits such that production efficiency thereof becomes excellent, and that convenience of a user can be improved.

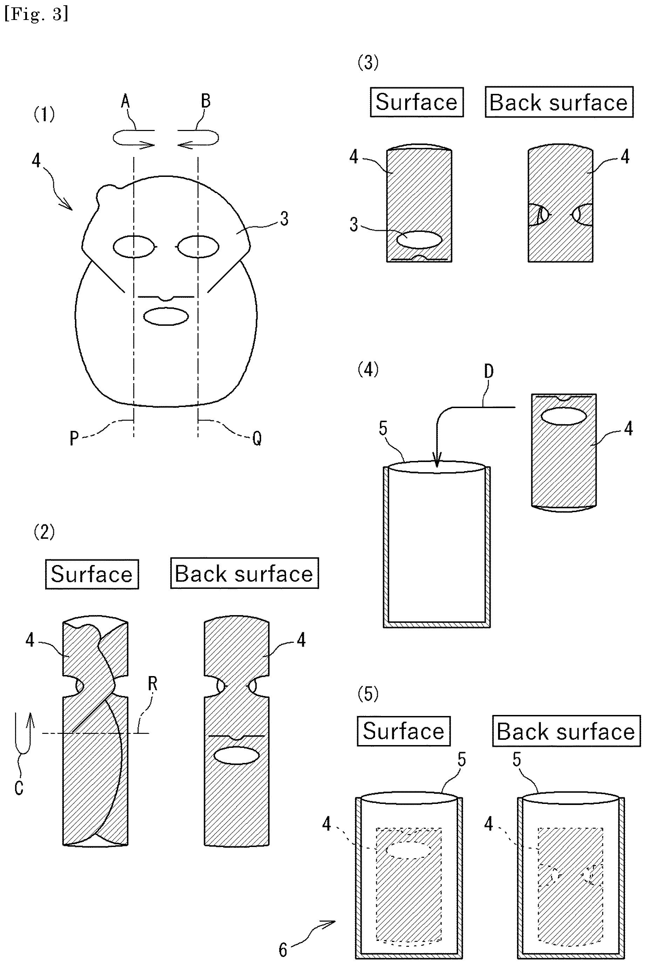

According to the embodiments shown in Fig. 2, the cosmetic sheet 4 is folded so that the release layer of the cosmetic sheet 4 is positioned at an inner surface of the folded cosmetic sheet 4, and the cosmetic sheet 4is accommodated in the storage container 5. However, the present invention is not limited only to the embodiments. The cosmetic sheet 4 of the present invention can be folded so that the release layer 3 is contacted with an inner surface of the storage container 5 as shown in the embodiments of Fig. 3.

Incidentally, Fig. 3 is a schematic explanation view showing another embodiment of orders for folding the cosmetic sheet 4 of the present invention, and another embodiment of orders for accommodating the cosmetic sheet 4 in the storage container. The cosmetic sheet 4 shown in Fig. 3 has a shape of a face mask.

In Fig. 3, the release layer 3 of the cosmetic sheet 4 of the present invention is shown by oblique lines. First, as shown in Fig. 3 (1), the cosmetic sheet 4 of the present invention is valley folded at the creases P, Q as shown by arrows A and B, respectively, to fold the cosmetic sheet 4 as shown in Fig. 3 (2). The folded cosmetic sheet 4 shown in Fig. 3 (2) is then valley folded at the crease R as shown by an arrow C, to fold the cosmetic sheet 4 as shown in Fig. 3 (3). In accordance with the above orders, the cosmetic sheet 4 of the present invention can be folded so that the release layer 3 is positioned at an outer surface of the folded cosmetic sheet 4.

The cosmetic sheet 4 of the present invention folded in the above is accommodated in the storage container 5 of the cosmetic sheet 4, for example, as shown by an arrow D of Fig. 3 (4), and thereby the cosmetic article 6 can be obtained as shown in Fig. 3 (5). After the cosmetic sheet 4 is accommodated in the storage container 5, an opening of the storage container 5 of the cosmetic sheet 4 can be sealed by, for example, thermal fusion or the like.

Incidentally, it is preferred that the type and the size of the storage container 5 of the cosmetic sheet 4 can be the same as those described above.

According to the embodiments of orders for folding the cosmetic sheet 4 and the embodiments of orders for accommodating the cosmetic sheet 4 in the storage container 5, which are shown in Fig. 3, the release layer 3 of the cosmetic sheet 4 can prevent the elastomer film of the cosmetic sheet 4 from adhering to the inner surface of the storage container 5. Accordingly, a user of the cosmetic sheet 4 can easily take out the cosmetic sheet 4 from the storage container 5. In addition, according to the embodiments shown in Fig. 3, since the cosmetic sheet 4 is folded so that the release layer 3 of the cosmetic sheet 4 is positioned at the inner surface of the folded cosmetic sheet 4, the cosmetic sheet 4 does not necessitate a separator. Accordingly, the cosmetic sheet 4 and the cosmetic article 6, which are shown in Fig. 3, are excellent in production efficiency, and can improve convenience of a user.

### EXAMPLES

Next, the present invention will be more specifically described in accordance with working examples. However, the present invention is not limited only to those examples.

### Example 1

A resin composition composed of 20% by mass of a low-density polyethylene commercially available from Tosoh Corporation under the trade name of Petrosen 212 and 80% by mass of an ethylene-octene elastomer commercially available from DuPont Elastomers Japan Co., Ltd. under the trade name of Engage was extruded to laminate with a nonwoven fabric composed of 40% by mass of rayon fibers and 60% by mass of a mixture of polyethylene terephthalate (PET) and polyethylene (PE) (thermally fusible fiber: split fiber made of polyethylene, content of thermally fusible fiber: 24.5% by mass) commercially available from Daiwabo Polytec Co., Ltd. under the trade name of DFS (SH) R5-70 (area weight: 70 g/m²), to give a laminated product in which an elastomer film having a thickness of 6 µm was formed on the surface of the nonwoven fabric. The laminated product obtained in the above was observed. As a result, it was confirmed that a part of the fibers constituting the nonwoven fabric of the laminated product was integrally bonded to the elastomer film.

Next, a silicone resin-based mold releasing agent was applied to the surface of the elastomer film of the laminated product obtained in the above by means of gravure coating, and the silicone resin mold releasing agent was dried to form a release layer, to give a cosmetic sheet. The thickness of the release layer of the cosmetic sheet obtained in the above was about 0.1 µm.

### Example 2

A urethane resin-based mold releasing agent was applied to a release paper, and dried to form an elastomer film.

A urethane resin-based binder (adhesive agent) was applied to the elastomer film obtained in the above in a dot-like manner. A nonwoven fabric composed of 40% by mass of rayon fibers and 60% by mass of a mixture of polyethylene terephthalate (PET) and polyethylene (PE) (thermally fusible fiber: split fiber made of polyethylene, content of thermally fusible fiber: 24.5 % by mass) commercially available from Daiwabo Polytec Co., Ltd. under the trade name of DFS (SH) R5-70 (area weight: 70 g/m²) was laminated on the surface of the elastomer film where the urethane resin-based binder was adhered, and the elastomer film was bonded to the nonwoven fabric, to give a laminated product.

Next, a urethane resin-based mold releasing agent was applied to the surface of the elastomer film of the laminated product obtained in the above by means of gravure coating, and the urethane resin-based mold releasing agent was dried to form a release layer, to give a cosmetic sheet. The thickness of the release layer of the cosmetic sheet obtained in the above was about 0.1 µm.

### Comparative example 1

A laminated product produced in the same manner as in Example 1 was used as a cosmetic sheet.

### Comparative example 2

A laminated product produced in the same manner as in Example 2 was used as a cosmetic sheet.

Next, as physical properties of the cosmetic sheet obtained in each example and each comparative example, blocking resistance, adhesiveness A and adhesiveness B were examined in accordance with the following methods.

### [Blocking resistance]

A cosmetic sheet (length: 100 mm, width: 150 mm) was folded in half so that the surface on which a release layer or an elastomer film was formed was positioned at an inside surface of the folded cosmetic sheet. Thereafter, the folded cosmetic sheet was put into an aluminum pouch, and an opening of the aluminum pouch was sealed, to give a cosmetic article. Two sets of the cosmetic article were produced.

The cosmetic article obtained in the above was placed in a thermostatic chamber having room temperature of 50°C. When one week or four weeks passed from placing of the cosmetic article in the thermostatic chamber, the cosmetic article was taken out from the thermostatic chamber. The cosmetic sheet was taken out from the cosmetic article by opening the cosmetic article, and the cosmetic sheet was observed with naked eyes.

As a result, blocking of the cosmetic sheets obtained in each of Examples 1 and 2 was not at all observed after two weeks and after 4 weeks passed from placing of the cosmetic article in the thermostatic chamber. To the contrary, the elastomer films of the cosmetic sheets obtained in each of Comparative Examples 1 and 2 were adhered to each other, and blocking was observed in those cosmetic sheets after two weeks and after 4 weeks passed from placing of the cosmetic article in the thermostatic chamber.

From the results mentioned above, it can be seen that the cosmetic sheets obtained in Examples 1 and 2 are excellent in blocking resistance as compared with the cosmetic sheet obtained in each of the comparative examples. From this fact, it is understood that a user of the cosmetic sheet obtained in those examples can easily spread the cosmetic sheet in use even when the cosmetic sheet is folded so that the release layer is positioned at an inner surface of the folded cosmetic sheet.

Next, the cosmetic sheets obtained in Examples 1 and 2 were cut with a cutting blade. As a result, the release layer of the cosmetic sheet was not adhered to the cutting blade, and the cosmetic sheet could be easily removed from the cutting blade.

### [Adhesiveness A]

An adhesive area of 50 mm in length and 30 mm in width of a commercially available masking tape was adhered to a face where the release layer or the elastomer film was formed at the end part of a short side having a length of 50 mm of the cosmetic sheet having a length of 100mm and a width of 50mm at room temperature. An area having a width of 20 mm of the masking tape where the cosmetic sheet was not adhered was bent toward the end part where the masking tape was adhered to the cosmetic sheet, and a digital force gauge commercially available from IMADA CO., LTD. under the product number of ZP-200N was attached to the bent area of the masking tape. A short side having a length of 50mm of the cosmetic sheet where the masking tape was not adhered was fixed with a clip.

Next, the digital force gauge was slowly pulled in the horizontal direction to determine the peel strength when the cosmetic sheet was peeled off. The peel strength was used as an index of adhesiveness.

As a result, the peel strength of the cosmetic sheet obtained in Example 1 was 0.1 N, and the peel strength of the cosmetic sheet obtained in Example 2 was 0.2 N. In contrast, the peel strength of each of the cosmetic sheets obtained in Comparative example 1 and Comparative example 2 was 0.7 N, respectively.

From the above results, it can be seen that the cosmetic sheets obtained in Examples 1 and 2 have little adhesiveness respectively since the peel strength of those cosmetic sheets is extremely low.

Next, after the determination of the peel strength, the face of the cosmetic sheet where the masking tape was peeled off was observed with naked eyes. As a result, no abnormality was found on the face from which the masking tape was peeled off in the cosmetic sheets obtained in Examples 1 and 2. On the other hand, roughness was observed on the face from which the masking tape was peeled off in the cosmetic sheets obtained in Comparative Examples 1 and 2.

From the above facts, since the surface of the release layer of the cosmetic sheets obtained in Examples 1 and 2 have hardly adhesiveness, and therefore, hardly adhere to a storage container such as an aluminum pouch, it can be seen that the cosmetic sheets obtained in Examples 1 and 2 are excellent in workability when the cosmetic sheet is accommodated in the storage container, and that a user can easily take out the cosmetic sheet from the cosmetic article in which the cosmetic sheet is accommodated in the storage container. In addition, since adhesiveness of the release layer of the cosmetic sheets obtained in Examples 1 and 2 is low, it can be seen that adhesion of the cosmetic sheets to a cutting blade can be prevented when the cosmetic sheet is cut into a predetermined shape with a cutting blade.

### [Adhesiveness B]

A cosmetic sheet having a length of 100 mm and a width of 50 mm was brought into close contact with a glass plate having a smooth surface so that the release layer or the elastomer film of the cosmetic sheet was contacted with the glass plate at room temperature. A load having a mass of about 6.8 kg was applied onto the cosmetic sheet for 5 minutes, and thereafter the cosmetic sheet was removed from the glass plate. The surface from which the glass plate was removed was observed with a digital microscope commercially available from KEYENCE CORPORATION under the product number of VHX -5000 at a magnification of 20 times.

As a result, no abnormality was found on the face of the cosmetic sheet from which the glass plate was removed in the cosmetic sheets obtained in Examples 1 and 2. On the other hand, removal of the elastomer film was observed on the face of the cosmetic sheet from which the glass plate was removed in the cosmetic sheets obtained in Comparative Examples 1 and 2.

From the above facts, since the surface of the release layer of the cosmetic sheets obtained in Examples 1 and 2 have hardly adhesiveness, and therefore, hardly adhere to a storage container, it can be seen that the cosmetic sheets are excellent in workability when the cosmetic sheet is accommodated in the storage container, and that a user can easily take out the cosmetic sheet from the cosmetic article in which the cosmetic sheet is accommodated in the storage container. In addition, since adhesiveness of the release layer of the cosmetic sheets obtained in Examples 1 and 2 is low, it can be seen that adhesion of the cosmetic sheets to a cutting blade can be prevented when the cosmetic sheet is cut into a predetermined shape with a cutting blade.

### INDUSTRIAL APPLICABILITY

The cosmetic sheet of the present invention is expected to be used in, for example, a face mask, a foot care sheet, a moisturizing sheet and the like.

### DESCRIPTION OF SYMBOLS

- 1: nonwoven fabric
- 2: elastomer film
- 3: release layer
- 4: cosmetic sheet
- 5: storage container for cosmetic sheet
- 6: cosmetic article

## Claims

1. A cosmetic sheet (4) comprising a nonwoven fabric (1) having stretching property, an elastomer film (2) and a release layer (3),
wherein the elastomer film (2) is laminated on the nonwoven fabric (1), and fibers included in the nonwoven fabric (1) are bonded to the elastomer film (2), **characterised in that** the release layer (3) is formed by applying and drying a mold releasing agent on the surface of the elastomer film (2) and
the release layer (3) is formed from at least one mold releasing agent selected from the group consisting of a silicone resin-based mold releasing agent, a urethane resin-based mold releasing agent, a wax-based mold releasing agent and a fluorocarbon resin-based mold releasing agent.

2. The cosmetic sheet (4) according to claim 1, wherein the nonwoven fabric (1) is a nonwoven fabric comprising thermally fusible fibers; the elastomer film (2) is an elastomer film (2) comprising a thermoplastic elastomer; and the thermally fusible fibers are fused into one body with the elastomer film (2).

3. A cosmetic article (6) comprising the cosmetic sheet (4) according to claim 1 or 2, and a storage container (5) for accommodating the cosmetic sheet (4), wherein the cosmetic sheet (4) is accommodated in the storage container (5).

4. The cosmetic article (6) according to claim 3, wherein the cosmetic sheet (4) is folded so that the release layer (3) of the cosmetic sheet (4) is positioned at an inner surface of the folded cosmetic sheet (4).

5. The cosmetic article (6) according to claim 3 or 4, wherein the cosmetic sheet (4) is folded so that the release layer (3) of the cosmetic sheet (4) is contacted with an inner surface of the storage container (5).

## Patentansprüche

1. Kosmetiktuch (4), das einen Vliesstoff (1) mit Dehneigenschaften, eine Elastomerfolie (2) und eine Trennschicht (3) umfasst, wobei die Elastomerfolie (2) auf den Vliesstoff (1) laminiert ist und die in dem Vliesstoff (1) enthaltenen Fasern an die Elastomerfolie (2) gebunden sind (2),
**dadurch gekennzeichnet, dass**
die Trennschicht (3) durch Applizieren und Trocknen eines Formtrennmittels auf der Oberfläche der Elastomerfolie (2) gebildet wird,
und dadurch, dass die Trennschicht (3) aus mindestens einem Formtrennmittel, das der Gruppe ausgewählt ist, die aus einem Formtrennmittel auf Silikonharzbasis, einem Formtrennmittel auf Urethanharzbasis, einem Formtrennmittel auf Wachsbasis und einem Formtrennmittel auf Basis eines fluorierten Kohlenwasserstoffharzes besteht, gebildet wird.

2. Kosmetiktuch (4) gemäß Anspruch 1, wobei der Vliesstoff (1) ein Vliesstoff ist, der thermisch schmelzbare Fasern umfasst, die Elastomerfolie (2) eine Elastomerfolie (2) ist, die ein thermoplastisches Elastomer umfasst, und die thermisch schmelzbaren Fasern mit der Elastomerfolie (2) zu einem Körper verschmolzen sind.

3. Kosmetikartikel (6), umfassend das Kosmetiktuch (4) gemäß Anspruch 1 oder Anspruch 2 und einen Aufbewahrungsbehälter (5) zur Unterbringung des Kosmetiktuchs (4), wobei das Kosmetiktuch (4) in dem Aufbewahrungsbehälter (5) untergebracht ist.

4. Kosmetikartikel (6) gemäß Anspruch 3, wobei das Kosmetiktuch (4) so gefaltet ist, dass sich die Trennschicht (3) des Kosmetiktuchs (4) an einer inneren Oberfläche des gefalteten Kosmetiktuchs (4) befindet.

5. Kosmetikartikel (6) gemäß Anspruch 3 oder Anspruch 4, wobei das Kosmetiktuch (4) so gefaltet ist, dass sich die Trennschicht (3) des Kosmetiktuchs (4) mit einer inneren Oberfläche des Aufbewahrungsbehälters (5) in Kontakt befindet.

## Revendications

1. Feuille cosmétique (4) comprenant un tissu non tissé (1) ayant une propriété d'étirement, un film élastomère (2) et une couche de démoulage (3),
dans laquelle le film élastomère (2) est laminé sur le tissu non tissé (1), et les fibres incluses dans le tissu non tissé (1) sont liées au film élastomère (2)
**caractérisée en ce que** la couche de démoulage (3) est formée par l'application et le séchage d'un agent de démoulage sur la surface du film élastomère (2),
et **en ce que** la couche de démoulage (3) est formée d'au moins un agent de démoulage choisi dans le groupe constitué par un agent de démoulage à base de résine de silicone, un agent de démoulage à base de résine d'uréthane, un agent de démoulage à base de cire et un agent de démoulage à base de résine de fluorocarbone.

2. Feuille cosmétique (4) selon la revendication 1, dans laquelle le tissu non tissé (1) est un tissu non tissé comprenant des fibres thermofusibles ; le film élastomère (2) est un film élastomère (2) comprenant un élastomère thermoplastique ; et les fibres thermofusibles sont fusionnées en un seul corps avec le film élastomère (2).

3. Article cosmétique (6) comprenant la feuille cosmétique (4) selon la revendication 1 ou la revendication 2, et un récipient de stockage (5) destiné à loger la feuille cosmétique (4), dans lequel la feuille cosmétique (4) est logée dans le récipient de stockage (5).

4. Article cosmétique (6) selon la revendication 3, dans lequel la feuille cosmétique (4) est pliée de telle sorte que la couche de démoulage (3) de la feuille cosmétique (4) est positionnée sur une surface intérieure de la feuille cosmétique (4) pliée.

5. Article cosmétique (6) selon la revendication 3 ou la revendication 4, dans lequel la feuille cosmétique (4) est pliée de manière à ce que la couche de démoulage (3) de la feuille cosmétique (4) soit en contact avec une surface intérieure du récipient de stockage (5).
